# EUROPÄISCHE PATENTSCHRIFT

(11) **EP 4 125 717 B1**
(45) Veröffentlichungstag und Bekanntmachung des Hinweises auf die Patenterteilung: **21.02.2024**
(21) Anmeldenummer: 21713916.1
(22) Anmeldetag: 18.03.2021
(51) Int. Cl.: A61F 2/16

(54) **INTRAOKULARLINSE**
INTRAOCULAR LENS
LENTILLE INTRAOCULAIRE

(30) Priorität: 26.03.2020 DE 102020108381
(43) Veröffentlichungstag der Anmeldung: 08.02.2023
(73) Patentinhaber: Carl Zeiss Meditec AG, 07745 Jena (DE)
(72) Erfinder: DAMM, Niklas, 10409 Berlin (DE)
(74) Vertreter: PATERIS Patentanwälte PartmbB
(86) Internationale Anmeldenummer: PCT/EP2021/056899
(87) Internationale Veröffentlichungsnummer: WO 2021/191036

(56) Entgegenhaltungen:
- WO-A1-94/28825

## Beschreibung

Die Erfindung betrifft eine Intraokularlinse.

Bei einer Kataraktbehandlung wird eine Intraokularlinse in den Kapselsack eines Auges eingesetzt. Die Intraokularlinse weist einen Optikkörper und eine Haptik auf, mittels der die Intraokularlinse in dem Kapselsack befestigt wird. Die Haptik hat die Aufgabe, den Optikkörper möglichst mittig und positionsstabil in dem Kapselsack zu halten. Ein Kapselsack hat bei einem Menschen typischerweise einen Durchmesser im Bereich von 9,75 mm bis 11,0 mm. Bei einem eher kleinen Kapselsack wird die Haptik eher stark komprimiert, wodurch es zu einem axialen Versatz des Optikkörpers kommen kann. Der axiale Versatz kann dazu führen, dass sich die Brechkraft des Optikkörpers, die vor der Kataraktbehandlung gewählt wurde, im Vergleich zu einer Intraokularlinse mit einer wenig komprimierten Haptik unkontrolliert und nicht prognostizierbar verändert.

EP 0 175 972 A1 offenbart eine Intraokularlinse mit einem einfahrbaren Bein. WO 94/28825 A1 offenbart eine Intraokularlinse zur postoperativen Repositionierung.

Aufgabe der Erfindung ist es daher, eine Intraokularlinse zu schaffen, bei der ein axialer Versatz der Intraokularlinse in einem Kapselsack eines Auges vermieden werden kann.

Die erfindungsgemäße Intraokularlinse weist einen Optikkörper und eine Haptik auf, die ein erstes Bauteil mit einem Einrastvorsprung und ein zweites Bauteil mit einer Einrastausnehmung aufweist, wobei der Einrastvorsprung und die Einrastausnehmung beabstandet voneinander angeordnet sind, wenn die Haptik in einem entspannten Zustand angeordnet ist, und eingerichtet sind, miteinander in Eingriff zu stehen, wenn ausgehend von dem entspannten Zustand die Haptik in Richtung zu dem Optikkörper hin via einen teilweise komprimierten Zustand der Haptik in einen vollständig komprimierten Zustand der Haptik bewegt ist.

Indem in dem komprimierten Zustand der Einrastvorsprung und die Einrastausnehmung in Eingriff stehen, ist die Haptik in dem komprimierten Zustand steifer als in dem entspannten Zustand oder in dem teilweise komprimierten Zustand. Dadurch kann sich die Haptik in dem komprimierten Zustand weniger leicht durchbiegen, wodurch ein axialer Versatz des Optikkörpers vermeidbar ist. Es ist denkbar, dass die Haptik so ausgeführt ist, dass sie sich bei einem relativ großen Kapselsack, wie beispielsweise mit einem Durchmesser länger als 10,0 mm, in dem teilweise komprimierten Zustand befindet und bei einem relativ kleinen Kapselsack, wie beispielweise mit einem Durchmesser kürzer als 10,0 mm, in dem komprimierten Zustand befindet.

Es ist bevorzugt, dass der Einrastvorsprung eine Vorsprungstirnfläche und die Einrastausnehmung eine Ausnehmungstirnfläche aufweist, die der Vorsprungstirnfläche zugewandt angeordnet ist und in dem komprimierten Zustand die Vorsprungstirnfläche kontaktiert. Wenn die Haptik ausgehend von dem entspannten Zustand in Richtung zu dem Optikkörper bewegt wird, wird von der Haptik eine Gegenkraft erzeugt, die umso höher ist, je näher die Haptik an dem Optikkörper angeordnet ist. Wenn die Ausnehmungstirnfläche die Vorsprungstirnfläche kontaktiert, wird dadurch erreicht, dass die Gegenkraft anschließend besonders stark ansteigt.

Es ist bevorzugt, dass die Intraokularlinse eine erste Bauteilstirnfläche, die benachbart zu dem Einrastvorsprung angeordnet ist, und eine zweite Bauteilstirnfläche aufweist, die benachbart zu der Einrastausnehmung angeordnet ist, wobei in dem komprimierten Zustand die erste Bauteilstirnfläche die zweite Bauteilstirnfläche kontaktiert. Wenn die Haptik ausgehend von dem entspannten Zustand in Richtung zu dem Optikkörper bewegt wird, wird von der Haptik eine Gegenkraft erzeugt, die umso höher ist, je näher die Haptik an dem Optikkörper angeordnet ist. Wenn die erste Bauteilstirnfläche die zweite Bauteilstirnfläche kontaktiert, wird dadurch erreicht, dass die Gegenkraft anschließend besonders stark ansteigt.

Die Haptik ist erfindungsgemäß einteilig ausgebildet. Dabei ist es erfindungsgemäß, dass die Haptik eine Haptikaussparung aufweist, die von dem ersten Bauteil und dem zweiten Bauteil begrenzt ist. Durch das Vorsehen der Haptikaussparung ist der Bereich der Haptik, der benachbart zu der Haptikaussparung angeordnet ist, schwächer als der Rest der Haptik ausgebildet, wodurch eine Verformung der Haptik bei einem Verschieben der Haptik von dem entspannten Bereich, via den teilweise komprimierten Zustand in den komprimierten Zustand hauptsächlich in diesem Bereich stattfinden wird.

Es ist zudem bevorzugt, dass in dem entspannten Zustand die Haptikaussparung in einer Ebene, deren Normale parallel zu einer optischen Achse des Optikkörpers ist, vollumfänglich von dem Material der Haptik begrenzt ist. Alternativ ist es bevorzugt, dass die Haptikaussparung in einer Ebene, deren Normale parallel zu der optischen Achse des Optikkörpers ist, nach außerhalb der Haptik kommuniziert. Die Haptikaussparung ist bevorzugt in einem Haptikbereich angeordnet, der sich ausgehend von dem Optikkörper bis zu maximal 30 % der Gesamtlänge der Haptik erstreckt. Dadurch wird erreicht, dass sich die Haptik hauptsächlich in der Nähe des Optikkörpers verformt.

Alternativ dazu, die Haptik einteilig auszubilden, kann gemäß einer nicht zu der Erfindung gehörenden Ausführungsform die Haptik mit zwei voneinander getrennten Teilen ausgebildet sein, die jeweils an dem Optikkörper befestigt sind und in dem entspannten Zustand und in dem teilweise komprimierten Zustand voneinander beabstandet angeordnet sind, wobei eins der zwei Teile das erste Bauteil aufweist und das andere der zwei Teile das zweite Bauteil aufweist. In dem komprimierten Zustand kontaktiert das eine der zwei Teile das andere der zwei Teile.

Es ist besonders bevorzugt, dass das eine der zwei Teile das erste Bauteil ist. Alternativ dazu ist es besonders bevorzugt, dass das andere der zwei Teile das zweite Bauteil ist.

Es ist bevorzugt, dass die Intraokularlinse einen ersten Bauteilvorsprung, von dem der Einrastvorsprung vorsteht, und/oder einen zweiten Bauteilvorsprung aufweist, in dem die Einrastausnehmung angeordnet ist. Insbesondere durch ein Wählen der Länge des ersten Bauteilvorsprungs in Richtung zu dem zweiten Bauteilvorsprung hin und/oder des zweiten Bauteilvorsprungs in Richtung zu dem ersten Bauteilvorsprung hin ist es besonders einfach möglich, einzustellen, wie weit die Haptik in Richtung zu dem Optikkörper hin zu bewegen ist, um in den komprimierten Zustand zu gelangen.

Es ist bevorzugt, dass der Einrastvorsprung eine erste Vorsprungseitenfläche aufweist und die Einrastausnehmung eine erste Ausnehmungseitenfläche aufweist, wobei in dem komprimierten Zustand die erste Vorsprungseitenfläche und die erste Ausnehmungseitenfläche einander zugewandt und benachbart zueinander angeordnet sind. Es ist zudem bevorzugt, dass der Einrastvorsprung eine zweite Vorsprungseitenfläche aufweist und die Einrastausnehmung eine zweite Ausnehmungseitenfläche aufweist, wobei in dem komprimierten Zustand die zweite Vorsprungseitenfläche und die zweite Ausnehmungseitenfläche einander zugewandt und benachbart zueinander angeordnet sind. Bei einem seitlichen Verschieben des Einrastvorsprungs gegen die Einrastausnehmung kann die erste Vorsprungseitenfläche gegen die erste Ausnehmungseitenfläche anstoßen oder die zweite Vorsprungseitenfläche kann gegen die zweite Ausnehmungseitenfläche anstoßen, wodurch das seitliche Verschieben begrenzt oder unterbunden werden kann.

Es ist bevorzugt, dass die Intraokularlinse zwei der Haptiken aufweist. Die zwei der Haptiken können jeweils an einem Bereich der Intraokularlinse befestigt sein, wobei die zwei Bereiche aneinander abgewandt angeordnet sind.

Es ist bevorzugt, dass die zwei der Haptiken symmetrisch zueinander bezüglich der optischen Achse des Optikkörpers sind.

Im Folgenden wird anhand der beigefügten schematischen Zeichnungen die Erfindung näher erläutert.
Figur 1 zeigt eine Draufsicht auf eine erste Ausführungsform einer erfindungsgemäßen Intraokularlinse.
Figur 2 zeigt ein Detail aus Figur 1.
Figur 3 zeigt eine perspektivische Ansicht eines Details der ersten Ausführungsform.
Figur 4 zeigt eine Draufsicht auf einen Teilbereich einer zweiten Ausführungsform der erfindungsgemäßen Intraokularlinse.

Figur 5 zeigt eine Draufsicht auf eine dritte Ausführungsform der Intraokularlinse, wobei die dritte Ausführungsform nicht zu der Erfindung gehört.

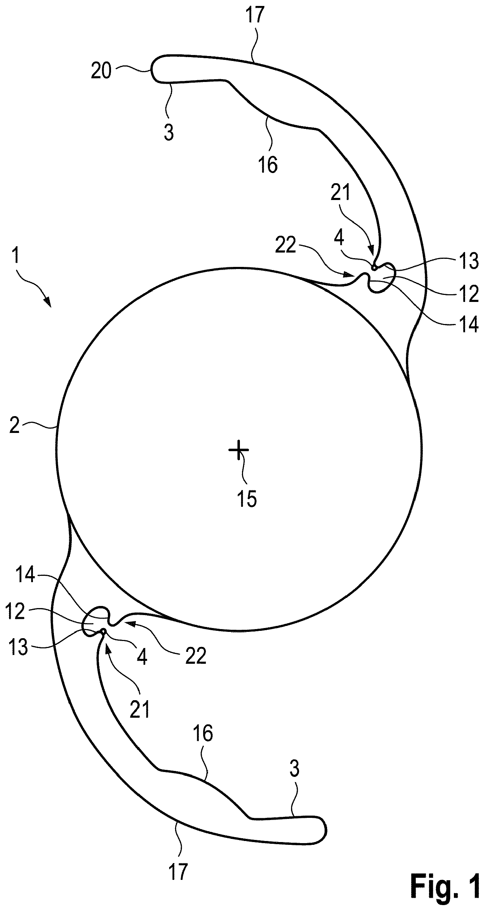

Wie es aus Figuren 1 bis 5 ersichtlich ist, weist eine Intraokularlinse 1 einen Optikkörper 2 und eine Haptik 3 auf, die ein erstes Bauteil 21 mit einem Einrastvorsprung 4 und ein zweites Bauteil 22 mit einer Einrastausnehmung 5 aufweist. Alternativ ist es möglich, dass das erste Bauteil 21 die Einrastausnehmung 5 aufweist und das zweite Bauteil 22 den Einrastvorsprung 4 aufweist. Der Einrastvorsprung 4 und die Einrastausnehmung 5 sind voneinander beabstandet angeordnet, wenn die Haptik 3 in einem entspannten Zustand der Haptik 3 angeordnet ist, wobei Figuren 1 bis 5 die Haptik 3 in dem entspannten Zustand zeigen. Zudem sind der Einrastvorsprung 4 und die Einrastausnehmung 5 eingerichtet, miteinander in Eingriff zu stehen, wenn die Haptik 3 ausgehend von dem entspannten Zustand in Richtung zu dem Optikkörper 2 hin via einen teilweise komprimierten Zustand der Haptik 3 in einen vollständig komprimierten Zustand der Haptik 3 bewegt ist. Wenn die Haptik 3 ausgehend von dem entspannten Zustand in Richtung zu dem Optikkörper 2 hin bewegt wird, gelangt demnach die Haptik 3 zuerst in den teilweise komprimierten Zustand, in dem der Einrastvorsprung 4 und die Einrastausnehmung 5 noch nicht miteinander in Eingriff stehen. Wird die Haptik 3 anschließend noch weiter in Richtung zu dem Optikkörper 3 hin bewegt, gelangt die Haptik 3 schließlich in den komprimierten Zustand, in dem der Einrastvorsprung 4 und die Einrastausnehmung 5 miteinander in Eingriff stehen. Figuren 1 bis 3 zeigen, dass das erste Bauteil 21 an einer Haptikinnenfläche 16 angeordnet sein kann, die dem Optikkörper 2 zugewandt angeordnet ist. Zusätzlich weist die Haptik 3 eine Haptikaußenfläche 17 auf, die der Haptikinnenfläche 16 abgewandt angeordnet ist und die dazu vorgesehen ist, wenn die Intraokularlinse 1 in einem Kapselsack eines Auges eingebracht ist, den Kapselsack zu kontaktieren.

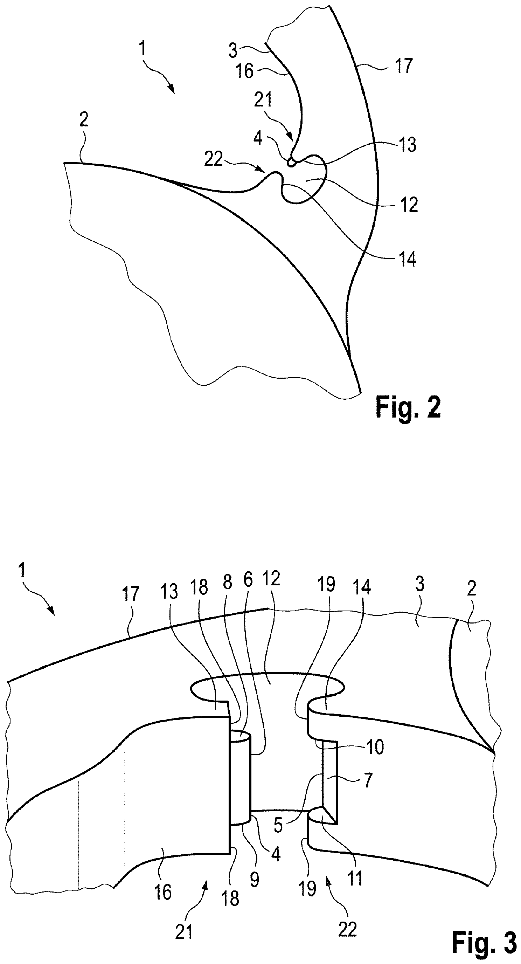

Figur 3 zeigt beispielhaft einen Einrastvorsprung 4 und eine Einrastausnehmung 5, wie sie in allen Ausführungsformen der Intraokularlinse 1 verbaut sein kann. Wie es aus Figur 3 ersichtlich ist, kann der Einrastvorsprung 4 in Richtung zu demjenigen des ersten Bauteils und des zweiten Bauteils, der die Einrastausnehmung 5 aufweist, hin sich verjüngen. Beispielsweise ist denkbar, dass dafür der Einrastvorsprung 4 die Form eines Halbzylinders hat, wie es beispielsweise in Figur 3 dargestellt ist. Aber auch andere Formen, wie beispielsweise ein Prisma oder eine Kalotte, sind denkbar.

Wie es aus Figur 3 ersichtlich ist, kann der Einrastvorsprung 4 eine Vorsprungstirnfläche 6 aufweisen und die Einrastausnehmung 5 kann eine Ausnehmungstirnfläche 7 aufweisen, die der Vorsprungstirnfläche 6 zugewandt angeordnet ist. In dem komprimierten Zustand kann die Ausnehmungstirnfläche 7 die Vorsprungstirnfläche 6 kontaktieren. Alternativ oder zusätzlich kann die Intraokularlinse 1 eine erste Bauteilstirnfläche 18, die benachbart zu dem Einrastvorsprung 4 angeordnet ist, und eine zweite Bauteilstirnfläche 19 aufweisen, die benachbart zu der Einrastausnehmung 5 angeordnet ist. In dem komprimierten Zustand kann die erste Bauteilstirnfläche 18 die zweite Bauteilstirnfläche 19 kontaktieren.

Figur 3 zeigt, dass der Einrastvorsprung 4 eine erste Vorsprungseitenfläche 8 aufweisen kann und die Einrastausnehmung 5 eine erste Ausnehmungseitenfläche 10 aufweisen kann, wobei in dem komprimierten Zustand die erste Vorsprungseitenfläche 8 und die erste Ausnehmungseitenfläche 10 einander zugewandt und benachbart zueinander angeordnet sind. Zudem zeigt Figur 3, dass der Einrastvorsprung 4 eine zweite Vorsprungseitenfläche 9 aufweisen kann und die Einrastausnehmung 5 eine zweite Ausnehmungseitenfläche 11 aufweisen kann, wobei in dem komprimierten Zustand die zweite Vorsprungseitenfläche 9 und die zweite Ausnehmungseitenfläche 11 einander zugewandt und benachbart zueinander angeordnet sind. Die Normalen der ersten Vorsprungseitenfläche 8, der zweiten Vorsprungseitenfläche 9, der ersten Ausnehmungseitenfläche 10 und der zweiten Ausnehmungseitenfläche 11 können parallel zu der optischen Achse 15 des Optikkörpers 2 angeordnet sein.

Figuren 1 bis 5 zeigen, dass die Intraokularlinse 1 einen ersten Bauteilvorsprung 13, von dem der Einrastvorsprung 4 vorsteht, und/oder einen zweiten Bauteilvorsprung 14 aufweisen kann, in dem die Einrastausnehmung 5 angeordnet ist.

In Figuren 1 bis 3 ist dargestellt, dass gemäß der ersten Ausführungsform der Intraokularlinse 1 die Haptik 3 einteilig ausgebildet ist. Die Haptik 3 weist eine Haptikaussparung 12 auf, die von dem ersten Bauteil 21 und dem zweiten Bauteil 22 begrenzt ist. In dem entspannten Zustand kommuniziert die Haptikaussparung 12 in einer Ebene, deren Normale parallel zu einer optischen Achse 15 des Optikkörpers 2 ist, nach außerhalb der Haptik 3. Es ist dabei denkbar, dass die Haptikaussparung 12 zwischen dem Einrastvorsprung 4 und der Einrastausnehmung 5 nach außerhalb der Haptik 3 kommuniziert.

Die Haptikaussparung 12 kann in einem Haptikbereich angeordnet sein, der sich ausgehend von dem Optikkörper 2 bis zu maximal 30 % der Gesamtlänge der Haptik 3 erstreckt. Die Gesamtlänge kann beispielsweise definiert sein, als die Länge einer Linie, die sich in der Mitte zwischen einer Haptikinnenfläche 16, die dem Optikkörper 2 zugewandt angeordnet ist, und einer Haptikaußenfläche 17, die dem Optikkörper 2 abgewandt angeordnet ist, ausgehend von dem Optikkörper 2 bis zu dem außenliegenden Längsende 20 der Haptik 3 erstreckt.

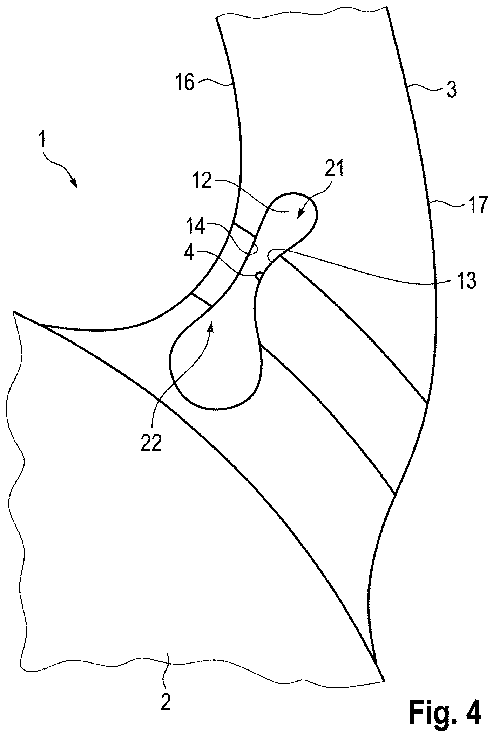

In Figur 4 ist dargestellt, dass gemäß der zweiten Ausführungsform der Intraokularlinse 1 die Haptik 3 einteilig ausgebildet ist. Dabei weist die Haptik 3 eine Haptikaussparung 12 auf, die von dem ersten Bauteil 21 und dem zweiten Bauteil 22 begrenzt ist. In dem entspannten Zustand ist die Haptikaussparung 12 in einer Ebene, deren Normale parallel zu einer optischen Achse 15 des Optikkörpers 2 ist, vollumfänglich von dem Material der Haptik 3 begrenzt. Die Haptikaussparung 12 kann in einem Haptikbereich angeordnet sein, der sich ausgehend von dem Optikkörper 2 bis zu maximal 30 % der Gesamtlänge der Haptik 3 erstreckt. Die Gesamtlänge kann beispielsweise definiert sein, als die Länge einer Linie, die sich in der Mitte zwischen einer Haptikinnenfläche 16, die dem Optikkörper 2 zugewandt angeordnet ist, und einer Haptikaußenfläche 17, die dem Optikkörper 2 abgewandt angeordnet ist, ausgehend von dem Optikkörper 2 bis zu dem außenliegenden Längsende 20 der Haptik 3 erstreckt.

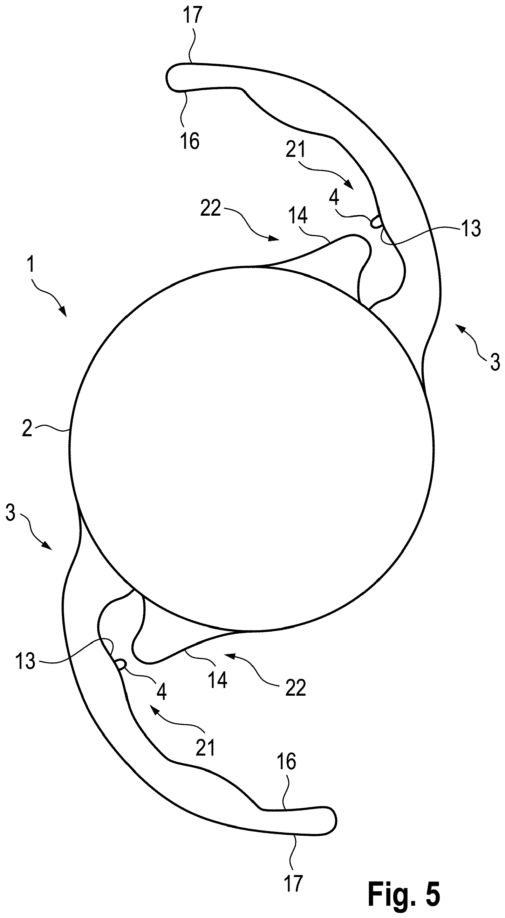

In Figur 5 ist dargestellt, dass gemäß der dritten und nicht zu der Erfindung gehörenden Ausführungsform der Intraokularlinse 1 die Haptik 3 mit zwei voneinander getrennten Teilen ausgebildet sein kann, die jeweils an dem Optikkörper 2 befestigt sind und in dem entspannten Zustand und in dem teilweise komprimierten Zustand voneinander beabstandet angeordnet sind, wobei eins der zwei Teile das erste Bauteil 21 aufweist und das andere der zwei Teile das zweite Bauteil 22 aufweist. Dabei ist denkbar, dass das andere der zwei Teile das zweite Bauteil 22 ist, wie es auch in Figur 5 dargestellt ist. Alternativ ist denkbar, dass das eine der zwei Teile das erste Bauteil 21 ist.

In Figuren 1 und 5 ist dargestellt, dass die Intraokularlinse 1 zwei der Haptiken 3 aufweisen kann. Die zwei der Haptiken 3 können an jeweils einem Bereich der Intraokularlinse 1 befestigt sein, wobei die zwei Bereiche aneinander abgewandt angeordnet sind. Zudem zeigen Figuren 1 und 5, dass die zwei der Haptiken 3 symmetrisch zueinander bezüglich der optischen Achse 15 des Optikkörpers 2 sein können.

### Bezugszeichenliste

1 Intraokularlinse
2 Optikkörper
3 Haptik
4 Einrastvorsprung
5 Einrastausnehmung
6 Vorsprungstirnfläche
7 Ausnehmungstirnfläche
8 erste Vorsprungseitenfläche
9 zweite Vorsprungseitenfläche
10 erste Ausnehmungseitenfläche
11 zweite Ausnehmungseitenfläche
12 Haptikaussparung
13 erster Bauteilvorsprung
14 zweiter Bauteilvorsprung
15 optische Achse
16 Haptikinnenfläche
17 Haptikaußenfäche
18 erste Bauteilstirnfläche
19 zweite Bauteilstirnfläche
20 Längsende
21 erstes Bauteil
22 zweites Bauteil

## Patentansprüche

1. Intraokularlinse mit einem Optikkörper (2) und einer Haptik (3), **dadurch gekennzeichnet, dass** die Haptik (3) ein erstes Bauteil (21) mit einem Einrastvorsprung (4) und ein zweites Bauteil (22) mit einer Einrastausnehmung (5) aufweist, wobei der Einrastvorsprung (4) und die Einrastausnehmung (5) beabstandet voneinander angeordnet sind, wenn die Haptik (3) in einem entspannten Zustand angeordnet ist, und eingerichtet sind, miteinander in Eingriff zu stehen, wenn ausgehend von dem entspannten Zustand die Haptik (3) in Richtung zu dem Optikkörper (2) hin via einen teilweise komprimierten Zustand der Haptik (3) in einen vollständig komprimierten Zustand der Haptik (3) bewegt ist, wobei die Haptik (3) einteilig ausgebildet ist und eine Haptikaussparung (12) aufweist, die von dem ersten Bauteil (21) und dem zweiten Bauteil (22) begrenzt ist.

2. Intraokularlinse gemäß Anspruch 1, wobei der Einrastvorsprung (4) eine Vorsprungstirnfläche (6) und die Einrastausnehmung (5) eine Ausnehmungstirnfläche (7) aufweist, die der Vorsprungstirnfläche (6) zugewandt angeordnet ist und in dem komprimierten Zustand die Vorsprungstirnfläche (6) kontaktiert.

3. Intraokularlinse gemäß Anspruch 1 oder 2, wobei die Intraokularlinse (1) eine erste Bauteilstirnfläche (18), die benachbart zu dem Einrastvorsprung (4) angeordnet ist, und eine zweite Bauteilstirnfläche (19) aufweist, die benachbart zu der Einrastausnehmung (5) angeordnet ist, wobei in dem komprimierten Zustand die erste Bauteilstirnfläche (18) die zweite Bauteilstirnfläche (19) kontaktiert.

4. Intraokularlinse gemäß einem der Ansprüche 1 bis 3, wobei in dem entspannten Zustand die Haptikaussparung (12) in einer Ebene, deren Normale parallel zu einer optischen Achse (15) des Optikkörpers (2) ist, vollumfänglich von dem Material der Haptik (3) begrenzt ist oder in der Ebene, deren Normale parallel zu der optischen Achse (15) ist, nach außerhalb der Haptik (3) kommuniziert.

5. Intraokularlinse gemäß einem der Ansprüche 1 bis 4, wobei die Haptikaussparung (12) in einem Haptikbereich angeordnet ist, der sich ausgehend von dem Optikkörper (2) bis zu maximal 30 % der Gesamtlänge der Haptik (3) erstreckt.

6. Intraokularlinse gemäß einem der Ansprüche 1 bis 5, wobei die Intraokularlinse (1) einen ersten Bauteilvorsprung (13), von dem der Einrastvorsprung (4) vorsteht, und/oder einen zweiten Bauteilvorsprung (14) aufweist, in dem die Einrastausnehmung (5) angeordnet ist.

7. Intraokularlinse gemäß einem der Ansprüche 1 bis 6, wobei der Einrastvorsprung (4) eine erste Vorsprungseitenfläche (8) aufweist und die Einrastausnehmung (5) eine erste Ausnehmungseitenfläche (10) aufweist, wobei in dem komprimierten Zustand die erste Vorsprungseitenfläche (8) und die erste Ausnehmungseitenfläche (10) einander zugewandt und benachbart zueinander angeordnet sind.

## Claims

1. Intraocular lens having an optical body (2) and a haptic element (3), **characterized in that** the haptic element (3) comprises a first component (21) with a latching projection (4) and a second component (22) with a latching recess (5), wherein the latching projection (4) and the latching recess (5) are spaced from one another when the haptic element (3) is in a relaxed state, and are designed to engage with one another when, from the relaxed state, the haptic element (3) is moved towards the optical body (2) into a fully compressed state of the haptic element (3) via a partially compressed state of the haptic element (3), wherein the haptic element (3) is formed in one piece and has a haptic cutout (12), which is delimited by the first component (21) and the second component (22).

2. Intraocular lens according to Claim 1, wherein the latching projection (4) has a projection end face (6) and the latching recess (5) has a recess end face (7), which faces towards the projection end face (6) and makes contact with the projection end face (6) in the compressed state.

3. Intraocular lens according to Claim 1 or 2, wherein the intraocular lens (1) has a first component end face (18), which is adjacent to the latching projection (4), and a second component end face (19), which is adjacent to the latching recess (5), wherein the first component end face (18) makes contact with the second component end face (19) in the compressed state.

4. Intraocular lens according to one of Claims 1 to 3, wherein, in the relaxed state, the haptic cutout (12) is delimited all around the periphery by the material of the haptic element (3) in a plane of which the normal is parallel to an optical axis (15) of the optical body (2), or communicates with outside the haptics element (3) in the plane of which the normal is parallel to the optical axis (15).

5. Intraocular lens according to one of Claims 1 to 4, wherein the haptic cutout (12) is arranged in a haptic region which extends from the optical body (2) to at most 30% of the total length of the haptic element (3).

6. Intraocular lens according to one of Claims 1 to 5, wherein the intraocular lens (1) has a first component projection (13), from which the latching projection (4) protrudes, and/or a second component projection (14), in which the latching recess (5) is arranged.

7. Intraocular lens according to one of Claims 1 to 6, wherein the latching projection (4) has a first projection side face (8) and the latching recess (5) has a first recess side face (10), wherein the first projection side face (8) and the first recess side face (10) face one another and are adjacent to one another in the compressed state.

## Revendications

1. Lentille intraoculaire comprenant un corps optique (2) et un système haptique (3), **caractérisée en ce que** le système haptique (3) comporte un premier composant (21) pourvu d'une saillie d'encliquetage (4) et un deuxième composant (22) pourvu d'un évidement d'encliquetage (5), la saillie d'encliquetage (4) et l'évidement d'encliquetage (5) étant disposés à distance l'un de l'autre lorsque le système haptique (3) est disposé dans un état détendu et étant conçus pour s'engager l'un avec l'autre lorsque le système haptique (3) est déplacé en direction du corps optique (2) à partir de l'état détendu, jusque dans un état complètement comprimé du système haptique (3) via un état partiellement comprimé du système haptique (3), le système haptique (3) étant formé d'une seule pièce et comportant un évidement haptique (12) qui est délimité par le premier composant (21) et le deuxième composant (22) .

2. Lentille intraoculaire selon la revendication 1, la saillie d'encliquetage (4) ayant une face d'extrémité de saillie (6) et l'évidement d'encliquetage (5) ayant une face d'extrémité d'évidement (7) qui est disposée face à la face d'extrémité de saillie (6) et entre en contact avec la face d'extrémité de saillie (6) à l'état comprimé.

3. Lentille intraoculaire selon la revendication 1 ou 2, la lentille intraoculaire (1) comportant une première face de composant frontale (18) qui est disposée de manière adjacente à la saillie d'encliquetage (4), et une deuxième face de composant frontale (19) qui est disposée de manière adjacente à l'évidement d'encliquetage (5), la première face de composant frontale (18) venant en contact avec la deuxième face de composant frontale (19) à l'état comprimé.

4. Lentille intraoculaire selon l'une des revendications 1 à 3, à l'état détendu l'évidement haptique (12) étant entièrement délimité par la matière du système haptique (3) dans un plan dont la normale est parallèle à un axe optique (15) du corps optique (2) ou communiquant avec l'extérieur du système haptique (3) dans le plan dont la normale est parallèle à l'axe optique (15).

5. Lentille intraoculaire selon l'une des revendications 1 à 4, l'évidement haptique (12) étant disposé dans une zone haptique qui s'étend depuis le corps optique (2) jusqu'à un maximum de 30 % de la longueur totale du système haptique (3).

6. Lentille intraoculaire selon l'une des revendications 1 à 5, la lentille intraoculaire (1) comportant une première saillie de composant (13), de laquelle la saillie d'encliquetage (4) fait saillie, et/ou une deuxième saillie de composant (14) dans laquelle l'évidement d'encliquetage (5) est ménagé.

7. Lentille intraoculaire selon l'une des revendications 1 à 6, la saillie d'encliquetage (4) comportant une première surface de saillie latérale (8) et l'évidement d'encliquetage (5) comportant une première surface d'évidement latérale (10), à l'état comprimé la première surface de saillie latérale (8) et la première surface d'évidement latérale (10) étant dirigées l'une vers l'autre et étant disposées de manière adjacente l'une à l'autre.
